# EUROPEAN PATENT APPLICATION

(11) **EP 3 981 256 A1**
(43) Date of publication of application: **13.04.2022**
(21) Application number: 20819386.2
(22) Date of filing: 05.06.2020
(51) Int. Cl.: A23C 9/152, A23L 33/125, A23L 33/135, C12N 1/20

(54) **COMPOSITION**

(30) Priority: 05.06.2019 JP 2019105521
(71) Applicant: Morinaga Milk Industry Co., Ltd., Minato-ku Tokyo 108-8384 (JP)
(72) Inventor: KUBO, Shutaro, Zama-shi, Kanagawa 252-8583 (JP); MURATA, Mai, Zama-shi, Kanagawa 252-8583 (JP); ODA, Hirotsugu, Zama-shi, Kanagawa 252-8583 (JP)
(74) Representative: Vossius & Partner Patentanwälte Rechtsanwälte mbB
(86) International application number: PCT/JP2020/022291
(87) International publication number: WO 2020/246583

(57) **Abstract**

An object of the present invention is to provide a technique for further promoting growth of bacteria belonging to the genus *Bifidobacterium.* By combining lactoferrin and/or lactoferrin hydrolyzate, and human milk oligosaccharides, a synergistic growth promoting effect of the bacteria belonging to the genus *Bifidobacterium* is achieved. Preferably, the human milk oligosaccharides include one or more selected from the group consisting of 2'-fucosyllactose, 3-fucosyllactose, and lactodifucotetraose. In addition, preferably, the bacteria belonging to the genus *Bifidobacterium* include one or more selected from the group consisting of *Bifidobacterium breve, Bifidobacterium infantis,* and *Bifidobacterium longum.*

## Description

### Technical Field

The present invention relates to a composition comprising lactoferrin and/or lactoferrin hydrolyzate, and human milk oligosaccharides.

### Background Art

Bacteria belonging to the genus *Bifidobacterium* (hereinafter also referred to as bifidobacteria) is known to predominate in the intestinal bacterial flora of infants, and this is thought to be closely related to the maintenance of infant health. Therefore, developing food that promotes the growth of bifidobacteria is important for maintaining the health of infants.

Lactoferrin is known as a milk protein and a factor that promotes the growth of bifidobacteria (Non Patent Literature 1), and bovine lactoferrin is widely used in powdered milk for nursing, supplements, etc. Furthermore, it has also been reported that a lactoferrin degradation product (lactoferrin-derived peptide) has an effect of promoting the growth of bifidobacteria (Non Patent Literature 2 and Patent Literature 1).

In addition, it has also been reported that human milk oligosaccharide (hereinafter also referred to as HMO), which is a general term for various types of oligosaccharides contained in human colostrums, promotes the growth of bifidobacteria (Patent Literature 2).

### Citation List

### Patent Literature

[PTL 1] US2015/139955A1
[PTL 2] US2012/294840A1

### Non Patent Literature

[NPL 1] Rahman, M.M. et. al., Int. J. Food Sci. Tech. 45:453-458, 2010
[NPL 2] H. Oda et. al., Appl. Environ. Microbiol., 79(6) 1843-1849, 2013

### Summary of Invention

### Technical Problem

An object of the invention is to provide a technique for further promoting the growth of bifidobacteria.

### Solution to Problem

As a result of diligent research to solve the above problem, the inventors of the invention have found that by combining lactoferrin and/or lactoferrin hydrolyzate, and human milk oligosaccharides, a synergistic growth promoting effect of the bacteria belonging to the genus *Bifidobacterium* is achieved, and have completed the invention.

That is, a first embodiment of the invention is a composition comprising human milk oligosaccharides and one or both of lactoferrin and/or lactoferrin hydrolyzate .

In the present embodiment, the mass ratio of the human milk oligosaccharides to lactoferrin and/or lactoferrin hydrolyzate is preferably 1/100000 or more and 1/10 or less.

In the embodiment, lactoferrin and/or lactoferrin hydrolyzate is preferably present in a content of 0.001% by mass or more and less than 100% by mass with respect to the whole composition.

In the embodiment, the composition comprises an amount of human milk oligosaccharides of 0.001% by mass or more and less than 100% by mass, with respect to the whole composition.

The human milk oligosaccharides in the embodiment preferably are selected from the group consisting of 2'-fucosyllactose, 3-fucosyllactose, lactodifucotetraose, and combinations thereof.

The composition of the embodiment can be preferably used for promoting growth of bacteria belonging to the genus *Bifidobacterium.* Here, the bacteria belonging to the genus *Bifidobacterium* are preferably selected from the group consisting of *Bifidobacterium breve, Bifidobacterium infantis, Bifidobacterium longum,* and combinations thereof.

Furthermore, a second embodiment of the invention is an composition formulated for oral use, wherein said comprosition comprises lactoferrin and/or lactoferrin hydrolyzate, human milk oligosaccharides, and bacteria belonging to the genus *Bifidobacterium.*

In the present embodiment, the bacteria belonging to the genus *Bifidobacterium* are preferably selected from the group consisting of *Bifidobacterium breve, Bifidobacterium infantis, Bifidobacterium longum,* and combinations thereof.

In the embodiment, the mass ratio of the human milk oligosaccharides to lactoferrin and/or lactoferrin hydrolyzate is preferably 1/100000 or more and 1/10 or less.

The human milk oligosaccharides in the embodiment preferably are selected from the group consisting of 2'-fucosyllactose, 3-fucosyllactose, lactodifucotetraose, and combinations thereof.

The composition formulated for oral use of the embodiment is preferably a nutritional composition for infants, and particularly preferably a modified milk for infants.

### Advantageous Effects of Invention

According to the invention, a composition which can promote the growth of bifidobacteria is provided. Such a composition can be formulated into a food or drink and pharmaceuticals as additives and the like. In addition, by further formulating the composition of the invention as an oral composition including bifidobacteria, it is expected that an effect of improving the intestinal bacterial flora can be obtained, which is useful for maintaining the health of eaters, especially infants.

### Brief Description of Drawings

Fig. 1 is a graph showing a growth promoting effect of 2'-fucosyllactose and lactoferrin on *Bifidobacterium breve* in Example 1.
Fig. 2 is a graph showing a growth promoting effect of 2'-fucosyllactose and lactoferrin on *Bifidobacterium infantis* in Example 2.
Fig. 3 is a graph showing a growth promoting effect of 2'-fucosyllactose and lactoferrin on *Bifidobacterium infantis* in Example 2.

### Description of Embodiments

Next, the invention will be described in detail. However, the invention is not limited to the following embodiments, and can be freely modified within the scope of the invention.

The composition of the first embodiment of the invention includes lactoferrin and/or lactoferrin hydrolyzate, and human milk oligosaccharides.

Lactoferrin is an iron-binding glycoprotein found in milk, tears, saliva, blood, etc. of a mammal such as sheep, goat, pig, mouse, buffalo, camel, yak, horse, donkey, llama, cow, and human.

The lactoferrin in the invention may be derived from any mammal, and is not particularly limited. However, from the viewpoint of content and availability, for example, lactoferrin derived from the milk of cow, human, and the like is preferable. The milk may be any of colostrum, transitional milk, normal milk, and late lactation milk.

Furthermore, the lactoferrin in the invention may be lactoferrin separated from skimmed milk, whey, etc., which are processed products of the aforementioned milk, by a conventional method (such as ion chromatography), recombinant lactoferrin produced from microorganisms, animal cells, transgenic animals, etc. by genetic manipulation, synthetic lactoferrin, or a mixture thereof. In addition, lactoferrin may be non-glycosylated or glycosylated. As such lactoferrin, commercially available lactoferrin produced on an industrial scale (for example, produced by Morinaga Milk Industry Co., Ltd.) may be used.

The metal content in the lactoferrin in the invention is not particularly limited, and any one or a combination of - apo-type lactoferrin obtained by deironing lactoferrin with hydrochloric acid, citric acid or the like; metalsaturated lactoferrin with a saturation of 100% or more obtained by chelating the apo-type lactoferrin with a metal such as iron, copper, zinc, and manganese; and metalpartially-saturated lactoferrin with a metal bonded at various saturations of less than 100% can be used.

Here, an example of a method for preparing lactoferrin (separation and purification of lactoferrin from a raw material such as milk) used in the present technique is shown below. However, the method is not limited thereto.

A bovine-derived dairy raw material is passed through a cation exchange column. This passing liquid is collected and is repeatedly passed through the column as appropriate. Deionized water is passed through the column, and saline is passed through the column to obtain an eluate of basic protein adsorbed on the cation exchange column. The eluate is collected by an ammonium sulfate precipitation method and is washed as appropriate. Collected precipitate is dissolved in deionized water, and this solution is filtered through an ultrafiltration membrane. Furthermore, by desalting and freeze-drying, powdered lactoferrin can be obtained.

More specifically, first, the column is filled with an ion exchanger, hydrochloric acid is passed through the column, and the ion exchanger is equilibrated by washing with water. Subsequently, skimmed milk having a pH of 6.9 cooled to 4°C is passed through the column, and a permeated liquid is collected and is passed through the column again in the same manner. Then, deionized water is passed through the column, and saline is passed through the column to obtain an eluate of basic protein adsorbed on the ion exchanger. Ammonium sulfate having a saturation of 80% is added to the eluate, and the protein is precipitated and centrifuged to collect the precipitate. The collected precipitate is washed with an ammonium sulfate solution having a saturation of 80%, dissolved by adding deionized water, and the obtained solution is desalted using an ultrafiltration membrane module and freeze-dried to obtain powdered bovine lactoferrin. In this way, bovine lactoferrin having a purity of 95% by mass or more can be obtained.

In lactoferrin, there are naturally gene mutations such as substitution, deletion, insertion, addition, and inversion of one or more bases at one or more positions depending on differences in species, genus, individual, etc., and mutations may also occur in amino acids of proteins encoded by genes with such mutations. The lactoferrin that can be used in the technique in the invention includes those having such mutations as long as the effects of the invention are not impaired.

In addition, the lactoferrin in the invention may also include a lactoferrin-treated product that has been heattreated, acid-treated, or alkaline-treated, as long as the effects of the invention are not impaired.

The lactoferrin hydrolyzate in the invention refers to those obtained by subjecting the aforementioned lactoferrin to a hydrolysis treatment.

Examples of the hydrolysis treatment include a method described in JP2012-235768A.

Specifically, the pH of the lactoferrin solution is adjusted to 2 to 4, preferably 2.5 to 3.5, particularly preferably to pH 3 with an acid such as hydrochloric acid, citric acid, and acetic acid before the lactoferrin solution is subjected to an enzymatic reaction treatment.

After adding an enzyme composition having chymosin in a desired amount to the pH-adjusted lactoferrin solution, the solution is stirred for 6 hours to 24 hours, preferably 12 to 18 hours while maintaining the temperature of the enzymatic reaction at 35 to 55°C, preferably 40 to 50°C, more preferably 42 to 48°C to hydrolyze lactoferrin.

Next, for example, the reaction solution is heated to 80°C and maintained for 10 minutes to inactivate the enzyme. Furthermore, preferably, an alkaline solution such as a sodium hydroxide solution is added to adjust the pH to 5 to 7, for example, to 6.

The pH-adjusted reaction solution (lactoferrin hydrolyzate) may remain as a solution; however, it is preferably powdered by freeze-drying or the like. In addition, as a lactoferrin decomposition product, those fractionated by chromatography, ultrafiltration or the like may also be used.

In the composition of the embodiment, the content of lactoferrin and/or lactoferrin hydrolyzate is preferably 0.001% by mass or more and less than 100% by mass, more preferably 0.005 to 75% by mass, further more preferably 0.01 to 50% by mass with respect to the whole composition.

The human milk oligosaccharide in the invention is not particularly limited as long as it is an oligosaccharide normally found in human milk. However, preferable examples include neutral human milk oligosaccharides such as 2'-fucosyllactose, 3-fucosyllactose, lactodifucotetraose, 2',3-difucosyllactose, lacto-N-triose II, lacto-N-tetraose, lacto-N-neotetraose, lacto-N -fucopentaose I, lacto-N-neofucopentaose, lacto-N-fucopentaose II, lacto-N-fucopentaose III, lacto-N-fucopentaose V, lacto-N-neofucopentaose V, lacto-N-difucohexaose I, lacto-N-difucohexaose II, 6'-galactosyl lactose, 3'-galactosyl lactose, lacto-N-hexaose, and lacto-N-neohexaose, and acidic human milk oligosaccharides such as 3'-sialyl lactose, 6'-sialyl lactose, 3-fucosyl-3'-sialyl lactose, and disialyllacto-N-tetraose. Of these, 2'-fucosyllactose, 3-fucosyllactose, and lactodifucotetraose are preferably, and 2'-fucosyllactose is particularly preferable, from the viewpoint of an effect of promoting the growth of bacteria belonging to the genus *Bifidobacterium.*

The human milk oligosaccharide in the invention may be a purified product or a mixture as long as the effects of the invention are not impaired. In addition, it may be one or more of the aforementioned human milk oligosaccharides.

In the composition of the embodiment, the content of the human milk oligosaccharides is preferably 0.001% by mass or more and less than 100% by mass, more preferably 0.005 to 75% by mass, further more preferably 0.01 to 50% by mass with respect to the whole composition.

Furthermore, in the composition of the embodiment, the mass ratio of the human milk oligosaccharides to lactoferrin and/or lactoferrin hydrolyzate is preferably 1/100000 or more and 1/10 or less, more preferably 1/10000 or more and 1/10 or less, further more preferably 1/1000 or more and 1/10 or less.

The composition of the invention can promote the growth of bacteria belonging to the genus *Bifidobacterium.*

Here, "promoting the growth" means to increase the number of bacteria belonging to the genus *Bifidobacterium* in the case where the composition of the invention is applied *in vivo* or *in vitro* as compared with the number in the case where the composition is not applied. The degree of such increase in the number of bacteria is not particularly limited; however, it is preferably 0.1% or more, more preferably 1% or more, further more preferably 5% or more greater than the number of bacteria belonging to the genus *Bifidobacterium* in the case where the composition of the invention is not applied.

Such an increase in the number of bacteria can be confirmed by, in addition to directly measuring the number of bacteria, for example, measuring the turbidity (absorbance) of a medium in which the bacteria have been cultured or the amount of short-chain fatty acids such as acetic acid and finding that the value is increased, or measuring the pH in the medium and finding that the value is decreased.

It has been known that each of lactoferrin and/or lactoferrin hydrolyzate and human milk oligosaccharides shows an effect of promoting the growth of bacteria belonging to the genus *Bifidobacterium.* However, as shown in Examples to be described later, by combining the two, the effect is synergistically exerted, and an excellent growth promoting effect of bacteria belonging to the genus *Bifidobacterium* is exhibited.

The bacteria belonging to the genus *Bifidobacterium* whose growth is promoted by the composition of the invention are not particularly limited, and examples thereof include *Bifidobacterium breve, Bifidobacterium infantis* (reclassified as *Bifidobacterium longum* subspecies *infantis*), *Bifidobacterium longum, Bifidobacterium bifidum, Bifidobacterium adolescentis, Bifidobacterium catenulatum, Bifidobacterium pseudocatenulatum, Bifidobacterium animalis, Bifidobacterium lactis,* and *Bifidobacterium pseudolongum.* Of these, one or more of *Bifidobacterium* breve, *Bifidobacterium infantis,* and *Bifidobacterium longum* are preferable.

The composition of the first embodiment of the invention may itself be in the form of food or drink, pharmaceuticals, etc., or may be formulated into a food or drink, pharmaceuticals and the like as an additive.

The route of ingestion (administration) of the composition of the embodiment may be oral or parenteral; however, it is usually oral. In addition, as parenteral ingestion (administration), rectal administration and the like can be mentioned.

When the composition of the first embodiment of the invention is to be orally ingested (administered), it is preferable that the composition further includes bacteria belonging to the genus *Bifidobacterium.*

That is, the second embodiment of the invention is an oral composition having lactoferrin and/or lactoferrin hydrolyzate, human milk oligosaccharides, and bacteria belonging to the genus *Bifidobacterium.*

The description of lactoferrin and/or lactoferrin hydrolyzate, and human milk oligosaccharides in this embodiment conforms to the description in the first embodiment described above.

In the composition of the second embodiment, the content of lactoferrin and/or lactoferrin hydrolyzate is preferably 0.001 to 5.0% by mass, more preferably 0.005 to 1.0% by mass, further more preferably 0.01 to 0.5% by mass with respect to the whole composition.

Moreover, in the composition of the second embodiment, the content of the human milk oligosaccharides is preferably 0.001 to 5.0% by mass, more preferably 0.005 to 1.0% by mass, further more preferably 0.01 to 0.5% by mass with respect to the whole composition.

These may usually be in the range of content when the composition is distributed as an oral composition.

The content of the composition of the second embodiment when it is orally ingested (administered) may be in the aforementioned range, or may be appropriately diluted. For example, the content of lactoferrin and/or lactoferrin hydrolyzate in the case of oral ingestion (administration) can be 0.0001 to 5.0% by mass with respect to the whole composition. In addition, the content of the human milk oligosaccharides in the case of oral ingestion (administration) can be 0.0001 to 5.0% by mass with respect to the whole composition.

In the embodiment, the bacteria belonging to the genus *Bifidobacterium* include at least viable cells, and preferably include 1.0 × 10⁶ cfu or more, more preferably 1.0 × 10⁷ cfu or more, further more preferably 2.0 × 10⁷ cfu or more, of live cells of bacteria belonging to the genus *Bifidobacterium* per 1mL of the oral composition. As long as live cells are present, dead cells can also be present.

Note that cfu refers to a colony forming unit. In the present description, for example, it can be the value when cultured at 38°C in a solid medium comprising 10% by mass of reduced skim milk powder.

These may usually be in the range of content when the composition is distributed as an oral composition.

Examples of the bacteria belonging to the genus *Bifidobacterium* to be formulated in the oral composition in the embodiment include those of the types described in the first embodiment. However, one or more of *Bifidobacterium breve, Bifidobacterium infantis,* and *Bifidobacterium longum* are preferable.

More specifically, as *Bifidobacterium breve, Bifidobacterium breve* M-16V can be mentioned. *Bifidobacterium breve* M-16V was internationally deposited with a deposit number of NITE BP-02622 under the Budapest Treaty at the National Institute of Technology and Evaluation, Patent Microorganisms Depository (NPMD) (Room No. 122, 2-5-8, Kazusakamatari, Kisarazu-shi, Chiba Prefecture, 292-0818), on January 26, 2018. For example, *"Bifidobacterium breve* M-16V" produced by Morinaga Milk Industry Co., Ltd., which is available as a commercially available product, may also be used.

Furthermore, more specifically, as *Bifidobacterium breve, Bifidobacterium breve* MCC1274 can also be mentioned. *Bifidobacterium breve* MCC1274 was internationally deposited with a deposit number of FERM BP-11175 under the Budapest Treaty at the National Institute of Advanced Industrial Science and Technology, Patent Microorganisms Depositary (which is currently the National Institute of Technology and Evaluation, International Patent Organism Depository (IPOD) (Room No. 120, 2-5-8, Kazusakamatari, Kisarazu-shi, Chiba Prefecture, 292-0818), on August 25, 2009.

More specifically, as *Bifidobacterium infantis, Bifidobacterium infantis* M-63 can also be mentioned. *Bifidobacterium infantis* M-63 was internationally deposited with a deposit number of NITE BP-02623 under the Budapest Treaty at NPMD on January 26, 2018.

More specifically, as *Bifidobacterium longum, Bifidobacterium longum* NITE BP-02621 (also known as BB536 or *Bifidobacterium longum* subsp. *Longum* ATCC BAA-999) can be used. *Bifidobacterium longum* BB536 was internationally deposited with a deposit number of NITE BP-02621 under the Budapest Treaty at the NPMD on January 26, 2018. The same bacterium, *Bifidobacterium longum* subsp. *Longum* ATCC BAA-999 (number: ATCC BAA-999) is available from American Type Culture Collection (ATCC: 10801 University Boulevard, Manassas, VA 20110, United States of America) as ATCC BAA-999 (see, for example, JP2012-223134A).

Bacteria specified by the above-exemplified bacterial names are not limited to strains that have been deposited or registered with predetermined institutions under the bacterial names (hereinafter, also referred to as "deposited strains" for convenience of explanation), and also include strains (also referred to as "derivative strains" or "derived strains") substantially equivalent thereto. In other words, the bacteria are not limited to the strains that have been deposited with the aforementioned depositary institutions with the aforementioned deposit numbers, and also include strains substantially equivalent thereto. For each bacterium, "a strain substantially equivalent to the aforementioned deposited strain" refers to a strain which belongs to the same species as the aforementioned deposited strain, has an effect of improving intestinal bacterial flora, preferably further has the same mycological properties as the deposited strain, and further the base sequence of the 16SrRNA gene of which has preferably 99.85% or more, more preferably 99.93% or more, further more preferably 100% identity with respect to the base sequence of the 16SrRNA gene of the deposited strain. For each bacterium, a strain substantially equivalent to the aforementioned deposited strain may be, for example, a derivative strain having the deposited strain as a parent strain. Examples of the derivative strains include strains bred from the deposited strains and strains naturally generated from the deposited strains. Examples of the breeding method include modification by a genetic engineering method and modification by a mutation treatment. Examples of the mutation treatment include irradiation with X-rays, irradiation with ultraviolet rays, and treatments with mutant agents such as N-methyl-N'-nitro-N-nitrosoguanidine, ethylmetanesulfonate, and methylmethanesulfonate. Examples of naturally generated strains from the deposited strains include strains that are naturally generated during the use of the deposited strains. Examples of such strains include mutant strains naturally generated by culturing (for example, subculturing) the deposited strains. The derivative strains may be constructed by one type of modification, or may be constructed by two or more types of modification.

For cells of the bacteria belonging to the genus *Bifidobacterium* that can be formulated into the oral composition of the embodiment, a commercially available product may be used, or those obtained by appropriate production may be used.

Furthermore, the cells of the bacteria belonging to the genus *Bifidobacterium* that can be formulated into the oral composition of the embodiment can be easily obtained by culturing the aforementioned bacteria belonging to the genus *Bifidobacterium.* A culturing method is not particularly limited as long as the bacteria belonging to the genus *Bifidobacterium* can grow. As the culturing method, for example, a method conventionally used for culturing bacteria belonging to the genus *Bifidobacterium* can be used as it is or after being appropriately modified. The culturing temperature may be, for example, 25 to 50°C, and is preferably 35 to 42°C. The culturing can be preferably carried out under anaerobic conditions, and for example, can be carried out under ventilation with an anaerobic gas such as carbon dioxide. In addition, the culturing can also be carried out under microaerobic conditions such as liquid stationary culture. The culturing can be carried out, for example, until the bacteria belonging to the genus *Bifidobacterium* grow to a desired degree.

A medium used for the culture is not particularly limited as long as the bacteria belonging to the genus *Bifidobacterium* can grow. For the medium, for example, a medium that is conventionally used for the culture of bacteria belonging to the genus *Bifidobacterium* can be used as it is, or after being appropriately modified. That is, as a carbon source, for example, saccharides such as galactose, glucose, fructose, mannose, cellobiose, maltose, lactose, sucrose, trehalose, starch, starch hydrolyzate, and molasses can be used depending on assimilability. As a nitrogen source, for example, ammonia, ammonium salts such as ammonium sulfate, ammonium chloride, and ammonium nitrate, and nitric acid salts can be used. Furthermore, as an inorganic salt, for example, sodium chloride, potassium chloride, potassium phosphate, magnesium sulfate, calcium chloride, calcium nitrate, manganese chloride, and ferrous sulfate can be used. In addition, organic components such as peptone, soybean flour, defatted soybean cake, meat extract, and yeast extract may also be used. Furthermore, as a medium that is conventionally used for the culture of bacteria belonging to the genus *Bifidobacterium,* specifically, reinforced clostridial medium, MRS medium (de Man, Rogosa, and Sharpe medium), mMRS medium (modified MRS medium), TOSP medium (TOS propionate medium), and TOSP Mup medium (TOS propionate mupirocin medium) may be mentioned.

Regarding the bacteria belonging to the genus *Bifidobacterium* that can be formulated into the oral composition of the embodiment, the cells thereof or fractions having the same can be used without particular limitation. That is, regarding the bacteria belonging to the genus *Bifidobacterium,* for example, the culture obtained by culturing may be used as it is, or the culture may be diluted or concentrated and used, or cells collected from the culture may be used. In addition, various additional operations such as heating and freeze-drying may be performed after culturing as long as the effect of improving the intestinal bacterial flora is not impaired. It is preferable that the additional operations are those such that the survivability of the cells is high. That is, examples of the bacteria belonging to the genus *Bifidobacterium* that can be formulated into the oral composition of the embodiment specifically include cultures of bacteria belonging to the genus *Bifidobacterium,* or cells collected from the cultures, and processed products thereof. Examples of the processed products include diluted products, concentrated products, and dried products. The cells are usually preferably used in a form that includes live cells. The cells may include, for example, live cells, or may be a mixture of live cells and dead cells.

The oral composition of the embodiment can promote the growth of bacteria belonging to the genus *Bifidobacterium* as described in the first embodiment. Here, the bacteria belonging to the genus *Bifidobacterium* to be proliferated are not limited to the bacteria belonging to the genus *Bifidobacterium* in the oral composition, and can also include bacteria belonging to the genus *Bifidobacterium* present in a digestive tract of an animal such as a human who has ingested.

Therefore, the oral composition of the embodiment can be used to improve the intestinal bacterial flora. Here, "improvement of bacterial flora" includes increasing the number of bacteria present in the intestinal bacterial flora of bacteria belonging to the genus *Bifidobacterium* or the existence ratio thereof. Further, "improvement of bacterial flora" may include increasing the existence ratio of other good bacteria in the intestinal bacterial flora or decreasing the existence ratio of bad bacteria in the intestinal bacterial flora, as long as the existence ratio of the bacteria belonging to the genus *Bifidobacterium* in the intestinal bacterial flora increases. Examples of other good bacteria include lactic acid bacteria. Examples of bad bacteria include *Clostridium perfringens,* bacteria belong to the genus *Salmonella, Staphylococcus aureus,* and enteropathogenic *Escherichia coli.*

The "existence ratio" can also be rephrased as "occupancy rate" with respect to the entire bacterial group detected in the intestinal bacterial flora.

The composition of the invention can be useful to a subject having a disease or pathological condition that can be prevented or ameliorated by the improvement of the intestinal bacterial flora, or to a subject having a disease or pathological condition caused by deterioration of the intestinal bacterial flora. For example, it can be used for intestinal regulation, immunomodulation, antiallergy, bacterial and/or viral infection protection, oxidative stress reduction, diarrhea prevention and/or amelioration, constipation prevention and/or amelioration, inflammatory enteric disease, colorectal cancer prevention, etc.

Another embodiment of the invention is the use of lactoferrin and/or lactoferrin hydrolyzate and human milk oligosaccharides in the production of a composition for promoting the growth of bacteria belonging to the genus *Bifidobacterium.*

Another embodiment of the invention is the use of lactoferrin and/or lactoferrin hydrolyzate and human milk oligosaccharides in promoting the growth of bacteria belonging to the genus *Bifidobacterium.*

Another embodiment of the invention is lactoferrin and/or lactoferrin hydrolyzate, and human milk oligosaccharides used for promoting the growth of bacteria belonging to the genus *Bifidobacterium.*

Another embodiment of the invention is a method of promoting the growth of bacteria belonging to the genus *Bifidobacterium,* including administering lactoferrin and/or lactoferrin hydrolyzate, and human milk oligosaccharides to an animal. Here, the animal is not particularly limited, and is usually a human.

The ingestion (administration) time of the composition of the invention is not particularly limited, and can be appropriately selected according to the condition of an administration subject.

The ingestion (administration) amount of the composition of the invention is appropriately selected according to the age, gender, condition, and other conditions of the ingestion (administration) subject.

The drug can be administered once a day or in several divided portions a day regardless of the amount and duration of ingestion (administration).

The composition of the invention is preferably in the form of food or drink.

The form and properties of the food or drink are not particularly limited as long as the effects of the invention are not impaired, and the food or drink can be produced by a conventional method using raw materials that are usually used for food or drink.

Embodiments of additives to be added to the food or drink are also included in the composition of the invention. Examples of such an embodiment include, for example, an additive to be added to collected breast milk or modified milk, and it is assumed that the milk after addition is to be ingested by a newborn baby or an infant.

Food and drink are usually taken orally; however, they are not limited thereto, and may be, for example, taken nasally, or may be taken by gastrostomy or intestinal fistula. For example, it is assumed that modified milk for infants to be described later, which is the composition of the invention, and breast milk to which the composition of the invention is added, are to be taken by newborns and infants through a nasal gastric feeding tube or the like.

The food or drink may be in any form such as liquid, paste, gel solid, and powder, and examples include tablet confectioneries; liquid foods (nutrient food for tube intake); flour products such as bread, macaroni, spaghetti, noodles, cake mix, frying powder, and bread crumbs; instant foods such as instant noodles, cup noodles, retort/cooked foods, cooked cans, microwave foods, instant soup/stew, instant miso soup/soup, canned soup, freeze dried foods, and other instant foods; processed agricultural products such as canned agricultural products, canned fruits, jams/marmalades, pickles, boiled beans, dried agricultural products, and cereals (processed grain products); processed seafood products such as canned seafoods, fish-flesh hams/sausages, seafood paste foods, seafood delicacies, and tsukudani; processed livestock products such as canned livestock/pastes and meat hams/sausages; milk/dairy products such as processed milk, milk drinks, yogurts, lactic beverages, cheese, ice creams, cream, and other dairy products; oils and fats such as butter, margarines, and vegetable oils; basic seasonings such as soy sauces, miso, sauces, processed tomato seasonings, mirins, and vinegars; complex seasonings/foods such as cooking mix, curry sauces, spicy soy sauces, dressings, mentsuyu, spices, and other complex seasonings; frozen foods such as frozen food ingredients, semi-cooked frozen foods, and cooked frozen food; confectioneries such as caramels, candies, chewing gums, chocolates, cookies, biscuits, cakes, pies, snacks, crackers, Japanese sweets, rice confectioneries, bean confectioneries, dessert confectioneries, jelly, and other confectioneries; favorite drinks such as carbonated drinks, natural fruit juices, fruit juice drinks, fruit juice- comprising soft drinks, pulp drinks, granule- comprising fruit drinks, vegetable-based drinks, soy milk, soy milk drinks, coffee drinks, tea drinks, powdered drinks, concentrated drinks, sports drinks, nutritional drinks, alcohol drinks, and other favorite drinks; commercially available foods such as baby foods, furikake, and ochazuke seaweed; nutritional compositions such as supplements and modified milk (including powdered milk, liquid milk, etc.); nutritional compositions for nursing (for infants) such as supplements for nursing and modified milk (including powdered milk, liquid milk, etc.) for nursing; enteric nutritional foods; functional foods (foods for specified health use, nutritional functional foods), etc.

Furthermore, examples of food or drink include milk for mothers during pregnancy and lactation (modified milk with a well-balanced combination of nutrients necessary for pregnancy and lactation), modified milk for later childhood and thereafter and modified milk for adults, nutrientcontrolling foods such as liquid foods for elderly people, dietary supplements, nutritional functional foods such as liquid foods, and foods for the sick (special purpose foods) .

Of these, a nutritional composition for infants is more preferable, and modified milk for infants is particularly preferable.

In addition, an embodiment of the food or drink may be feed. Examples of the feed include pet food, livestock feed, fish breeding feed, etc.

The form of the feed is not particularly limited. For example, the feed may include cereals such as corn, wheat, barley, rye and milo; vegetable oil meals such as soybean oil meal, rapeseed oil meal, coconut oil meal and linseed oil meal; brans such as wheat bran, rice bran, and defatted rice bran; production meals such as corn gluten meal and corn jam meal; animal or fish-derived feeds such as fish meal, skim milk powder, whey, yellow grease and tallow; yeasts such as torula yeast and brewer's yeast; mineral material feeds such as tribasic calcium phosphate and calcium carbonate; oils and fats; monomeric amino acids; saccharides, etc.

When the composition of the invention is in the form of food or drink (including feed), it can be provided and sold as a food or drink with an indication of use for growing bifidobacteria in the intestine.

Such an act of "indication" includes all the acts intended for publicizing the above-described application to consumers, and as long as the indication is an expression which may allow recall and analogy of the application, such expressions all correspond to the act of "indication" of the invention, irrespective of the purpose of indication, the content of indication, the object or medium of indication, and the like.

Furthermore, it is preferable that the "indication" is implemented by an expression from which consumers can directly recognize the above-described application. Specifically, acts of transferring, handing over, displaying and importing an article related to food or drink or an article packaging that describes the above-described application for transfer or handover; acts of describing the above-described application in advertisements, price lists or transaction documents related to an article and then displaying or distributing, or describing the application in the information containing these as the contents and providing the information by an electromagnetic (the Internet or the like) method; and the like may be mentioned.

On the other hand, regarding the content of the indication, the indication is preferably an indication approved by the government or the like (for example, an indication or the like approved under various institutions established by the government and put in a manner based on the approval). Furthermore, it is preferable that the content of such an indication is attached to a packaging, a container, a catalog, a pamphlet, an advertising material at a selling site such as POP, to documents other than those, or the like.

Furthermore, examples of the "indication" also include indications of a health food, a functional food, an enteric nutritional food, a food for special use, a health functional food, a special health food, a nutritional functional food, a functionality-indicated food, a quasi-drug, or the like. Particularly among these, indications approved by Consumers Affairs Agency, for example, indications approved by the institutions related to special health foods, nutritional functional foods, or functionality-indicated foods, or institutions similar to these, or the like may be mentioned. Specific examples include an indication of a special health food, an indication of a conditional special health food, an indication to the effect that the article may affect the structure or function of the body, an indication regarding a reduction of a disease risk, and an indication of the functionality based on a scientific basis. More specifically, typical examples include an indication that the article is a special health food (particularly an indication for health use) defined in Cabinet Office Ordinance on Permitting Special Use Labeling and the like as stipulated in the Health Promotion Act (August 31, 2009, Cabinet Office Ordinance No. 57), and indications similar to this.

Examples of such indications include "for those who want to increase bifidobacteria", "for improvement of intestinal flora", "for the health of infants' tummy" and the like.

When the composition of the invention is in the form of a drug, the route of administration thereof may be oral or parenteral; however, it is preferably oral. In addition, as parenteral ingestion (administration), rectal administration and the like can be mentioned.

Regarding the form of the drug, it can be appropriately formulated into a desired dosage form according to an administration method. For example, in the case of oral administration, it is possible to formulate solid preparations such as powder, granules, tablets, and capsules; and liquid preparations such as solutions, syrups, suspensions, and emulsions. Furthermore, in the case of parenteral administration, it is possible to formulate suppositories, ointment, injections, and the like.

During formulation, components generally used for formulation, such as an excipient, a pH adjuster, a colorant, and a corrigent, may be used. It is also possible to use other medicinal ingredients and prebiotics against bacteria belonging to the genus *Bifidobacterium* which are already known or to be found in the future in combination.

In addition, formulation can be carried out by a known method as appropriate depending on the dosage form. During formulation, a formulation carrier may be blended and formulated as appropriate.

Examples of excipients include sugar derivatives such as lactose, sucrose, glucose, mannitol, and sorbitol; starch derivatives such as corn starch, potato starch, α-starch, dextrin, and carboxymethyl starch; cellulose derivatives such as crystalline cellulose, hydroxypropyl cellulose, hydroxypropyl methyl cellulose, carboxymethyl cellulose, and carboxymethyl cellulose calcium; gum arabic; dextran; pullulan; silicate derivatives such as light anhydrous silicic acid, synthetic aluminum silicate, and magnesium aluminometasilicate; phosphate derivatives such as calcium phosphate; carbonate derivatives such as calcium carbonate; and sulfate derivatives such as calcium sulfate.

Examples of binders include gelatin; polyvinylpyrrolidone; macrogol and the like, in addition to the excipients.

Examples of disintegrants include cellulose derivatives or chemically modified starch such as sodium croscarmellose, sodium carboxymethyl starch, and crosslinked polyvinylpyrrolidone, in addition to the excipients.

Examples of lubricants include talc; stearic acid; metallic stearates such as calcium stearate, and magnesium stearate; colloidal silica; waxes such as bee gum, and spermaceti; boric acid; glycol; carboxylic acids such as fumaric acid and adipic acid; carboxylic acid sodium salts such as sodium benzoate; sulfates such as sodium sulfate; leucine; lauryl sulfates such as sodium lauryl sulfate, and magnesium lauryl sulfate; silicic acids such as silicic acid anhydride, and silicic acid hydrate; and starch derivatives.

Examples of stabilizers include paraoxybenzoic acid esters such as methylparaben and propylparaben; alcohols such as chlorobutanol, benzyl alcohol, and phenylethyl alcohol; benzalkonium chloride; acetic anhydride; and sorbic acid.

Examples of corrigents and flavoring agents include sweeteners, acidulants, flavors, etc.

As for carriers to be used in the case of liquid preparations for oral administration, a solvent such as water may be mentioned.

The timing of ingesting the drug of the invention is not particularly limited, and may be, for example, before meals, after meals, between meals, before bedtime, etc.

### [Examples]

The invention will be described in more detail below with reference to Examples. However, the invention is not limited to these Examples.

### <Example 1> Examination of the effects of human milk oligosaccharides and lactoferrin on the proliferative activity of Bifidobacterium breve

### (1) Preparation of medium and reagent

### (i) Preparation of MRS medium

DifcoMRS (BD) was dissolved in purified water as described in a package insert and sterilized at 115°C for 15 minutes. L-Cysteine hydrochloride (Wako) was dissolved in purified water, filtered and sterilized, and was aseptically added to MRS medium cooled to room temperature after sterilization to have a final concentration of 0.05% by mass.

### (ii) Preparation of glucose-free MRS medium

A reagent was dissolved in purified water with the composition shown in Table 1 and sterilized at 115°C for 15 minutes. L-Cysteine hydrochloride (Wako) was dissolved in purified water, filtered and sterilized, and was aseptically added to MRS medium cooled to room temperature after sterilization to have a final concentration of 0.05% by mass.

**[Table 1]**

| Composition | Amount |
|---|---|
| Polyoxyethylene Sorbitan Monooleate (Nacalai Tesque) | 1.25 g |
| Bacto Beef Extract (BD) | 12.5 g |
| BBL Polypeptone (BD) | 12.5 g |
| Bacto Yeast Extract (BD) | 6.25 g |
| Potassium Dihydrogen Phosphate (Kokusan Chemical) | 2.5 g |
| Sodium Acetate (Wako) | 6.25 g |
| Diammonium Hydrogen Citrate (Wako) | 2.5 g |
| Magnesium Sulfate Heptahydrate (Kokusan Chemical) | 0.5 g |
| Manganese Sulfate (II) Pentahydrate (Wako) | 0.0625 g |
| Purified Water | 900 mL |

### (iii) Preparation of human milk oligosaccharide solution and lactoferrin solution

Human milk oligosaccharide (2'-fucosyllactose (2'-FL) (Carbosynth)) and lactoferrin (produced by Morinaga Milk Industry Co., Ltd., iron content 15.8 mg/100 g) were dissolved in purified water, respectively. They were filtered and sterilized after dissolution.

### (2) Preparation of pre-culture solution

150 µL of a pre-culture solution of *Bifidobacterium breve* M-16V was added to 3 mL of MRS medium prepared in (1) (i), and was anaerobically cultured at 37°C for 16 hours. After culturing, the cells were separated from the culture solution by centrifuging at 1500 × g for 5 minutes. The culture solution was removed, and the cells were resuspended in 3 mL of glucose-free MRS medium prepared in (1) (ii).

### (3) Evaluation of growth promoting effect

The pre-culture solution prepared in (2) was added to the glucose-free MRS medium prepared in (1) (ii) to have a final concentration of 1 v/v%. Further, the 2'-fucosyllactose (2'-FL) solution and the lactoferrin solution prepared in (1) (iii) were respectively added to have the final concentrations shown in Table 2, and were dispensed into a 96-well plate at 200 µL/well.

1.0 mg/mL is 0.1% by mass when converted to mass percentage.

Then, the cells were anaerobically cultured at 37°C for 24 hours. After culturing, turbidity was measured at a wavelength of 600 nm using a microplate reader (Corona Electric) to evaluate the growth of bifidobacteria.

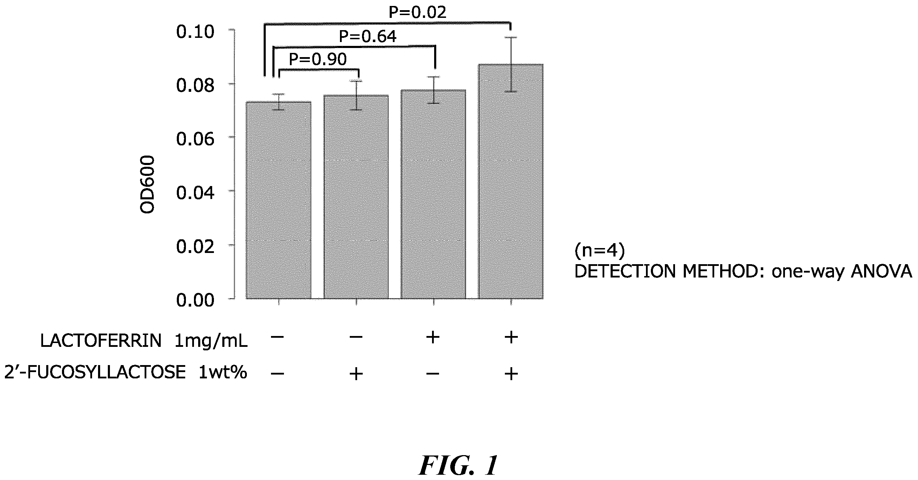

The results are shown in Table 2 and Fig. 1. When cultured in the coexistence of 2'-fucosyllactose and lactoferrin, significant growth of *Bifidobacterium breve* M-16V was observed compared with a control (2'-fucosyllactose and lactoferrin not added).

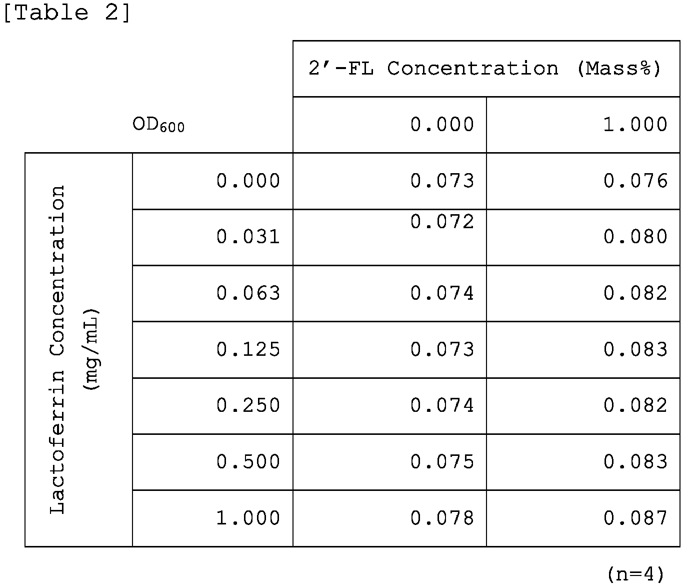

### <Example 2> Examination of the effects of human milk oligosaccharides and lactoferrin on the proliferative activity of Bifidobacterium infantis

The same operation as in Example 1 was carried out except that the bifidobacteria were replaced with *Bifidobacterium infantis* M-63, and the growth was evaluated.

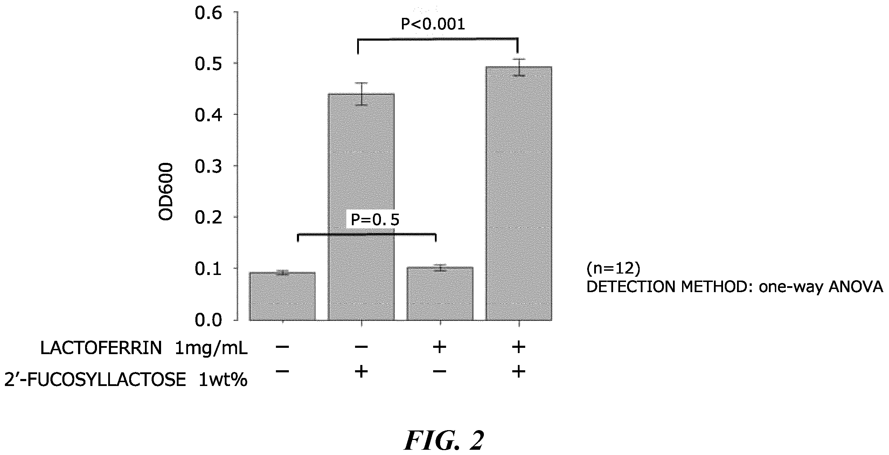

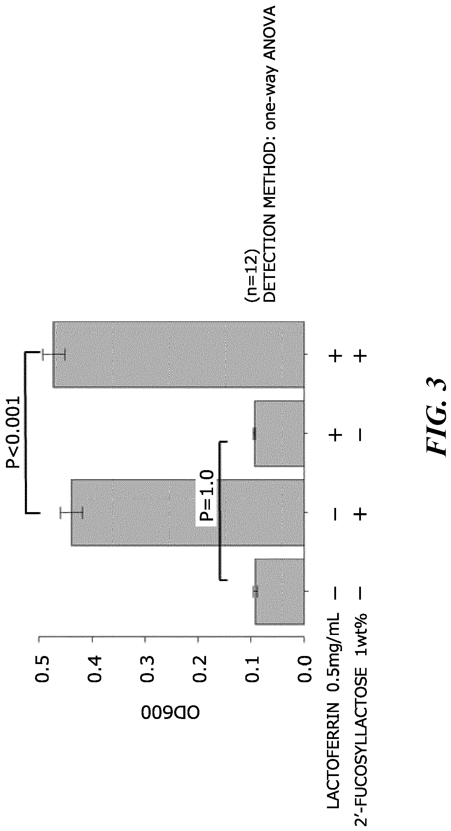

The results are shown in Table 3 and Figs. 2 and 3. When cultured in the coexistence of 2'-fucosyllactose and lactoferrin, a tendency of growth of *Bifidobacterium infantis* M-63 was observed compared with the control (2'-fucosyllactose and lactoferrin not added). Moreover, when cultured in the coexistence of 2'-fucosyllactose and lactoferrin, significant growth of *Bifidobacterium infantis* M-63 was observed compared with a group to which 2'-fucosyllactose was added and lactoferrin was not added.

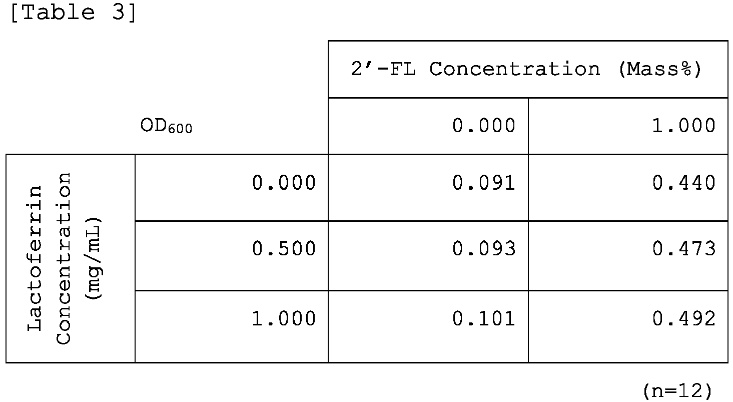

### <Example 3> Examination of the effects of human milk oligosaccharides and lactoferrin on the proliferative activity of Bifidobacterium longum

The same operation as in Example 1 was carried out except that the *bifidobacteria* were replaced with *Bifidobacterium longum* BB536, and the growth was evaluated.

The results are shown in Table 4. When cultured in the coexistence of 2'-fucosyllactose and lactoferrin, a tendency of growth of *Bifidobacterium longum* BB536 was observed compared with the control (2'-fucosyllactose and lactoferrin not added).

**[Table 4]**

| | | |
|---|---|---|
| 2'-FL Concentration (Mass%) | 0.000 | 1.000 |
| Lactoferrin Concentration (mg/mL) | 0.000 | 1.000 |
| OD₆₀₀ | 0.063 | 0.071 |

| | | |
|---|---|---|
| (n=2) | | |

### <Example 4> Examination of the effects of human milk oligosaccharides and lactoferrin on the proliferative activity of Bifidobacterium breve

The same operation as in Example 1 was carried out except that the *bifidobacteria* were replaced with *Bifidobacterium breve* MCC1274, and the growth was evaluated.

The results are shown in Table 5. When cultured in the coexistence of 2'-fucosyllactose and lactoferrin, a tendency of growth of *Bifidobacterium breve* MCC1274 was observed compared with the control (2'-fucosyllactose and lactoferrin not added).

**[Table 5]**

| | | |
|---|---|---|
| 2'-FL Concentration (Mass%) | 0.000 | 1.000 |
| Lactoferrin Concentration (mg/mL) | 0.000 | 1.000 |
| OD₆₀₀ | 0.085 | 0.111 |

| | | |
|---|---|---|
| (n=2) | | |

様式第4　　　　　　　　　　　　　　　　　　　　　　　　　　　　　　　　　　　　　　　　　　　国際
特許手続上の微生物の寄託の
国際的承認に関するブダペスト条約
下記国際寄託当局によってブダペスト条約に
基づく規則7．1に従い発行される
原寄託についての受託証
BUDAPEST TREATY ON THE INTERNATIONAL
RECOGNITION OF THE DEPOSIT OF MICROORGANISMS
FOR THE PURPOSES OF PATENT PROCEDURE
RECEIPT IN THE CASE OF AN ORIGINAL DEPOSIT
issued pursuant to Rule 7.1 by the
INTERNATIONAL DEPOSITARY AUTHORITY
identified at the bottom of this page

寄託者　氏名（名称）　森永乳業株式会社　様

DEPOSITOR　Name　Morinaga Milk Industry Co., Ltd.

住所　〒108-8384
東京都港区芝五丁目33番1号

Address　33-1, Shiba 5-chome, Minato-ku, Tokyo 1088384 Japan

I．微生物の表示　IDENTIFICATION OF THE MICROORGANISM
識別の表示：Identification reference given by the DEPOSITOR: | 受託番号：Accession number given by the INTERNATIONAL DEPOSITARY AUTHORITY：
Bifidobacterium longum BB536 | NITE　BP－ 02621
II．科学的性質及び分類学上の位置　SCIENTIFIC DESCRIPTION AND/OR PROPOSED TAXONOMIC DESIGNATION
I 欄の微生物には、次の事項が記載されていた。：The microorganism identified under I above was accompanied by:
■ 科学的性質　a scientific description
■ 分類学上の位置　a proposed taxonomic designation
III．受領及び受託　RECEIPT AND ACCEPTANCE
本国際寄託当局は、　2018 年 1 月 26日（原寄託日）に受領した I 欄の微生物を受託した。
This International Depositary Authority accepts the microorganism identified under I above, which was received by it
on　January 26, 2018　(date of the original deposit).
IV．移管請求の受領　RECEIPT OF REQUEST FOR CONVERSION
本国際寄託当局は、I 欄の微生物を　　年　　月　　日（国内寄託日）に受託しており、
ブダペスト条約に基づく国際寄託への移管請求を　　年　　月　　日に受領した。
The microorganism identified under I above was received by this International Depositary Authority
on　　　　(date of the original deposit) and a request to convert the original deposit to a deposit under the
Budapest Treaty was received by it on　　　　(date of receipt of request for conversion).
V．国際寄託当局　INTERNATIONAL DEPOSITARY AUTHORITY
独立行政法人製品評価技術基盤機構 特許微生物寄託センター
名称：　NITE Patent Microorganisms Depositary
Name：　National Institute of Technology and Evaluation
センター長　山崎　秀司
Shuji Yamazaki, Director General
住所：　〒292-0818 千葉県木更津市かずさ鎌足2-5-8 122号室
Address：　#122, 2-5-8 Kazusakamatari, Kisarazu-shi, Chiba 292-0818, Japan
発行日：2018 年 2 月 7 日
Date:　February 7, 2018

様式第4　　　　　　　　　　　　　　　　　　　　　　　　　　　　　　　　　　　　　　　国際
特許手続上の微生物の寄託の
国際的承認に関するブダペスト条約
BUDAPEST TREATY ON THE INTERNATIONAL
RECOGNITION OF THE DEPOSIT OF MICROORGANISMS
FOR THE PURPOSES OF PATENT PROCEDURE
下記国際寄託当局によってブダペスト条約に
基づく規則7．1に従い発行される
RECEIPT IN THE CASE OF AN ORIGINAL DEPOSIT
issued pursuant to Rule 7.1 by the
原寄託についての受託証
INTERNATIONAL DEPOSITARY AUTHORITY
identified at the bottom of this page
寄託者　氏名（名称）　森永乳業株式会社　様
DEPOSITOR　Name　Morinaga Milk Industry Co., Ltd.
住所　〒 108-8384
東京都港区芝五丁目33番1号
Address　33-1, Shiba 5-chome, Minato-ku, Tokyo 1088384 Japan
I．微生物の表示 | IDENTIFICATION OF THE MICROORGANISM
識別の表示： Identification reference given by the
DEPOSITOR:
Bifidobacterium breve M-16V | 受託番号： Accession number given by the
INTERNATIONAL DEPOSITARY AUTHORITY:
NITE　BP－ 02622
II．科学的性質及び分類学上の位置 | SCIENTIFIC DESCRIPTION AND/OR PROPOSED
TAXONOMIC DESIGNATION
I 欄の微生物には、次の事項が記載されていた。： The microorganism identified under I above was accompanied by:
■　科学的性質　a scientific description
■　分類学上の位置　a proposed taxonomic designation
III．受領及び受託 | RECEIPT AND ACCEPTANCE
本国際寄託当局は、　2018 年 1 月 26日（原寄託日）に受領した I 欄の微生物を受託した。
This International Depositary Authority accepts the microorganism identified under I above, which was received by it
on　January 26, 2018　(date of the original deposit).
IV．移管請求の受領 | RECEIPT OF REQUEST FOR CONVERSION
本国際寄託当局は、I 欄の微生物を　年　月　日（国内寄託日）に受託しており、
ブダペスト条約に基づく国際寄託への移管請求を　年　月　日に受領した。
The microorganism identified under I above was received by this International Depositary Authority
on　(date of the original deposit) and a request to convert the original deposit to a deposit under the
Budapest Treaty was received by it on　(date of receipt of request for conversion).
V．国際寄託当局 | INTERNATIONAL DEPOSITARY AUTHORITY
独立行政法人製品評価技術基盤機構 特許微生物寄託センター
名称：　NITE Patent Microorganisms Depositary
Name：　National Institute of Technology and Evaluation
センター長　山崎　秀司
Shuji Yamazaki, Director General
住所：　〒292-0818 千葉県木更津市かずさ鎌足2-5-8 122号室
Address：　#122, 2-5-8 Kazusakamatari, Kisarazu-shi, Chiba 292-0818, Japan
発行日： 2018 年 2 月 7 日
Date:　February 7, 2018

様式第4　　　　　　　　　　　　　　　　　　　　　　　　　　　　　　　　　　　　　　　　　　　国際
特許手続上の微生物の寄託の
国際的承認に関するブダペスト条約
下記国際寄託当局によってブダペスト条約に
基づく規則7．1に従い発行される
原寄託についての受託証
BUDAPEST TREATY ON THE INTERNATIONAL
RECOGNITION OF THE DEPOSIT OF MICROORGANISMS
FOR THE PURPOSES OF PATENT PROCEDURE
RECEIPT IN THE CASE OF AN ORIGINAL DEPOSIT
issued pursuant to Rule 7.1 by the
INTERNATIONAL DEPOSITARY AUTHORITY
identified at the bottom of this page

寄託者　氏名（名称）　森永乳業株式会社　様

DEPOSITOR　Name　Morinaga Milk Industry Co., Ltd.

住所　〒108-8384
東京都港区芝五丁目33番1号

Address　33-1, Shiba 5-chome, Minato-ku, Tokyo 1088384 Japan

I．微生物の表示　IDENTIFICATION OF THE MICROORGANISM
識別の表示：Identification reference given by the
DEPOSITOR:
Bifidobacterium infantis M-63 | 受託番号：Accession number given by the
INTERNATIONAL DEPOSITARY AUTHORITY:
NITE　BP－02623
II．科学的性質及び分類学上の位置　SCIENTIFIC DESCRIPTION AND/OR PROPOSED
TAXONOMIC DESIGNATION
I欄の微生物には、次の事項が記載されていた。：The microorganism identified under I above was accompanied by:
■　科学的性質　a scientific description
■　分類学上の位置　a proposed taxonomic designation
III．受領及び受託　RECEIPT AND ACCEPTANCE
本国際寄託当局は、　2018 年 1 月 26日（原寄託日）に受領したI欄の微生物を受託した。
This International Depositary Authority accepts the microorganism identified under I above, which was received by it
on　January 26, 2018　(date of the original deposit).
IV．移管請求の受領　RECEIPT OF REQUEST FOR CONVERSION
本国際寄託当局は、I欄の微生物を　年　月　日（国内寄託日）に受託しており、
ブダペスト条約に基づく国際寄託への移管請求を　年　月　日に受領した。
The microorganism identified under I above was received by this International Depositary Authority
on　(date of the original deposit) and a request to convert the original deposit to a deposit under the
Budapest Treaty was received by it on　(date of receipt of request for conversion).
V．国際寄託当局　INTERNATIONAL DEPOSITARY AUTHORITY
独立行政法人製品評価技術基盤機構 特許微生物寄託センター
名称：　NITE Patent Microorganisms Depositary
Name：　National Institute of Technology and Evaluation
センター長　山崎　秀司
Shuji Yamazaki, Director General
住所：　〒292-0818 千葉県木更津市かずさ鎌足2-5-8 122号室
Address：　#122, 2-5-8 Kazusakamatari, Kisarazu-shi, Chiba 292-0818, Japan
発行日：2018 年 2 月 7 日
Date:　February 7, 2018

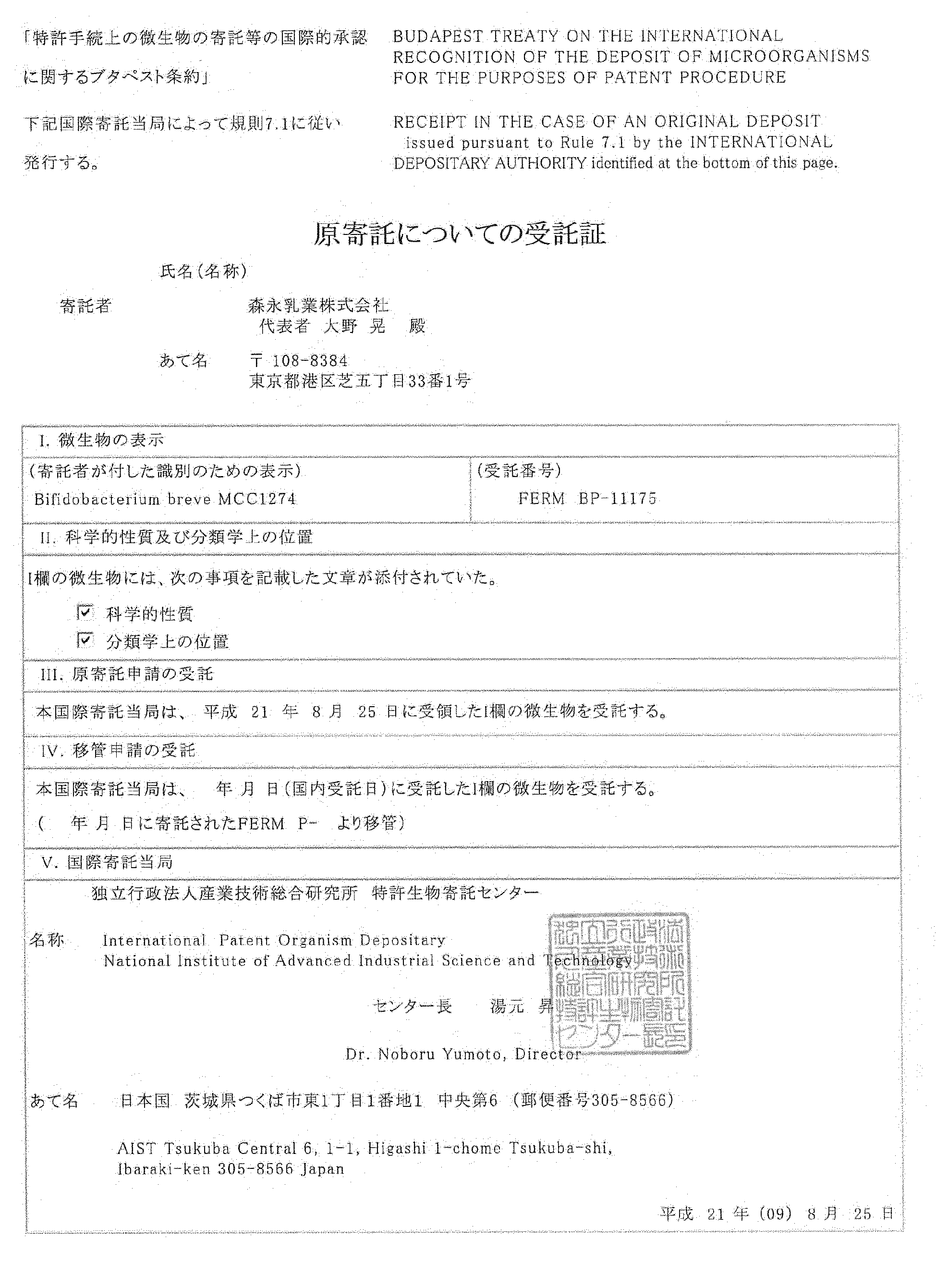
「特許手続上の微生物の寄託等の国際的承認
に関するブダペスト条約」

下記国際寄託当局によって規則7.1に従い
発行する。

BUDAPEST TREATY ON THE INTERNATIONAL
RECOGNITION OF THE DEPOSIT OF MICROORGANISMS
FOR THE PURPOSES OF PATENT PROCEDURE

RECEIPT IN THE CASE OF AN ORIGINAL DEPOSIT
issued pursuant to Rule 7.1 by the INTERNATIONAL
DEPOSITARY AUTHORITY identified at the bottom of this page.

原寄託についての受託証

氏名(名称)
寄託者　森永乳業株式会社
代表者　大野　晃　殿
あて名　〒 108-8384
東京都港区芝五丁目33番1号

I. 微生物の表示
(寄託者が付した識別のための表示) | (受託番号)
Bifidobacterium breve MCC1274 | FERM BP-11175
II. 科学的性質及び分類学上の位置
I欄の微生物には、次の事項を記載した文章が添付されていた。
☑ 科学的性質
☑ 分類学上の位置
III. 原寄託申請の受託
本国際寄託当局は、 平成 21 年 8 月 25 日に受領したI欄の微生物を受託する。
IV. 移管申請の受託
本国際寄託当局は、　年 月 日(国内受託日)に受託したI欄の微生物を受託する。
(　年 月 日に寄託されたFERM P-　より移管)
V. 国際寄託当局
独立行政法人産業技術総合研究所　特許生物寄託センター
名称　International Patent Organism Depositary
National Institute of Advanced Industrial Science and Technology
センター長　湯元　昇
Dr. Noboru Yumoto, Director
あて名　日本国　茨城県つくば市東1丁目1番地1　中央第6 (郵便番号305-8566)
AIST Tsukuba Central 6, 1-1, Higashi 1-chome Tsukuba-shi,
Ibaraki-ken 305-8566 Japan
平成 21 年 (09) 8 月 25 日

## Claims

1. A composition comprising lactoferrin and/or lactoferrin hydrolyzate and human milk oligosaccharides.

2. The composition according to claim 1, wherein the mass ratio of the human milk oligosaccharides to lactoferrin and/or lactoferrin hydrolyzate is 1/100000 or more and 1/10 or less.

3. The composition according to claim 1 or 2, wherein lactoferrin and/or lactoferrin hydrolyzate is comprised in a content of 0.001% by mass or more and less than 100% by mass with respect to the whole composition.

4. The composition according to any one of claims 1 to 3, wherein the human milk oligosaccharides are comprised in a content of 0.001% by mass or more and less than 100% by mass with respect to the whole composition.

5. The composition according to any one of claims 1 to 4, wherein the human milk oligosaccharides include one or more selected from the group consisting of 2'-fucosyllactose, 3-fucosyllactose, and lactodifucotetraose.

6. The composition according to any one of claims 1 to 5, which is used for promoting growth of bacteria belonging to the Genus Bifidobacterium.

7. The composition according to claim 6, wherein the bacteria belonging to the Genus Bifidobacterium include one or more selected from the group consisting of Bifidobacterium breve, Bifidobacterium infantis, and Bifidobacterium longum.

8. An oral composition comprising lactoferrin and/or lactoferrin hydrolyzate, human milk oligosaccharides, and bacteria belonging to the Genus Bifidobacterium.

9. The oral composition according to claim 8, wherein the bacteria belonging to the Genus Bifidobacterium include one or more selected from the group consisting of Bifidobacterium breve, Bifidobacterium infantis, and Bifidobacterium longum.

10. The oral composition according to claim 8 or 9, wherein the mass ratio of the human milk oligosaccharides to lactoferrin and/or lactoferrin hydrolyzate is 1/100000 or more and 1/10 or less.

11. The oral composition according to any one of claims 8 to 10, wherein the human milk oligosaccharides include one or more selected from the group consisting of 2'-fucosyllactose, 3-fucosyllactose, and lactodifucotetraose.

12. The oral composition according to any one of claims 8 to 11, which is modified milk for infants.
